# NEW EUROPEAN PATENT SPECIFICATION

(11) **EP 2 311 412 B2**
(45) Date of publication and mention of the opposition decision: **11.10.2017**
(45) Mention of the grant of the patent: 05.09.2012
(21) Application number: 10184636.8
(22) Date of filing: 11.12.2001
(51) Int. Cl.: A61F 2/915

(54) **Stent having helical elements**
Stent mit spiralförmigen Elementen
Stent à éléments en hélice

(30) Priority: 11.12.2000 US 254688 P
(43) Date of publication of application: 20.04.2011
(62) Divisional of application: 01992086.7
(73) Proprietor: OrbusNeich Medical, Inc., Ft Lauderdale, FL 33309 (US)
(72) Inventor: Addonizio, Scott, J., Fort Lauderdale, FL 33315 (US); Camp, David, L., Jr., Hillsboro Beach, FL 30062 (US); Becker, Gary, J., Tucson, AZ 85718 (US); Pazienza, John, D., Pompano Beach, FL 33060 (US)
(74) Representative: Jilderda, Anne Ayolt

(56) References cited:
- EP-A1- 0 806 190
- EP-A1- 0 827 725
- EP-A1- 0 876 806
- EP-A1- 0 884 029
- EP-A1- 0 983 753
- EP-A1- 1 020 166
- EP-A1- 1 025 812
- EP-A1- 1 027 870
- EP-A2- 0 540 290
- EP-A2- 0 686 379
- EP-A2- 0 968 689
- WO-A1-00/13611
- WO-A1-00/30563
- WO-A1-00/45742
- WO-A1-00/71053
- WO-A1-94/17754
- WO-A1-95/31945
- WO-A1-98/20810
- WO-A1-99/39660
- WO-A1-03/017870
- WO-A2-95/26695
- DE-U1- 297 008 689
- US-A- 5 772 864
- US-A- 5 913 897
- US-A- 6 132 460
- The 9th Kurashiki PTCA Live Demonstration Course Data book, Society of Chugoku interventional Cardiology; Kurashiki Central Hospital; Kurashiki, Okayama, published on February 23, 2000

## Description

### Field of the Invention

The present invention relates to a balloon expandable stent comprising at least one helical segment, and comprising a plurality of adjacent cylindrical elements connected to one another by a plurality of connecting elements, in which the cylindrical elements have cylindrical axes that are collinear with the stent's cylindrical axis and each cylindrical element includes a plurality of first circumferential segments, each first circumferential segment extending between two connecting elements and having a number of linear portions, wherein said number is exactly five, and a plurality of second circumferential segments, each second circumferential segment extending between two connecting elements and resembling generally an S-shaped structure having a number of linear portions, wherein said number is exactly three. In particular, the present invention relates to stents having helical elements.

### Description of Related Art

Stents are prosthetic devices that are implanted in the lumen of a vessel inside the body to provide support for the vessel's wall. Structural support from stents is particularly important in angioplasty procedures. Typically, stents are implanted within a vessel system to reinforce vessels that are partially occluded, collapsing, weakened, or abnormally dilated. More generally, stents can be used inside any physiological conduit or duct including, for example, arteries, veins, bile ducts, the urinary tract, alimentary tracts, the tracheobronchial tree, a cerebral aqueduct or the genitourinary system. Stents may be used in both humans and animals.

There are typically two types of stents: self expanding stents and balloon expandable stents. Self expanding stents automatically expand once they are released and assume a deployed, expanded state. A balloon expandable stent is expanded using an inflatable balloon catheter. The balloon is inflated to plastically deform the stent. Balloon expandable stents may be implanted by mounting the stent in an unexpanded or crimped state on a balloon segment of a catheter. The catheter, after having the crimped stent placed thereon, is inserted through a puncture in a vessel wall and moved through the vessel until it is positioned in the portion of the vessel that is in need of repair. The stent is then expanded by inflating the balloon catheter against the inside wall of the vessel. Specifically, the stent is plastically deformed by inflating the balloon so that the diameter of the stent is increased and remains at an increased state. In some situations, the vessel in which the stent is implanted may be dilated by the stent itself when the stent is expanded.

The Palmaz-Schattmtm stent, which is disclosed in the Handbook of Coronary Stents by Patrick W. Serruys et al. (Martin Dunitz, LTD 1998), is an example of a balloon expandable stent that had been implanted in hundreds of thousands of patients. The Palmaz-Schatztm stent, like other known stents, has certain limitations. These include, but are not limited to: (i) low stent-to-vessel ratio uniformity, (ii) comparative rigidity of the stent in a crimped as well as deployed state, and (iii) limited flexibility making delivery and placement in narrow vessels difficult. Stent-to-vessel ratio generally refers to the degree that the vessel wall is supported by the stent in its expanded state and preferably should be uniform throughout the length of the stent. Furthermore because the Palmaz-Schatztm stent consists of one or more bridges that connect a number of consecutively slotted tubes, there are a number of bare areas in the vessel after the expansion of the stent. These shortfalls are common to many stents. Id. at 36.

An expandable stent of the type described in the opening paragraph is known from European patent application EP 1.020.166 (Cottone et al.)..). This known stent comprises an expandable stent comprised of a plurality of helical segments. In one embodiment, the stent is generally cylindrical in shape, having a cylindrical axis, and comprises a first and second set of helical segments. The helical segments in the first set are substantially parallel and have a first pitch forming a first helical angle with respect to the cylindrical axis. The helical segments in the second set are also generally parallel to each other and form a second pitch that differs from the first pitch, thereby forming a second helical angle with respect to the cylindrical axis. In an alternative embodiment, the stent comprises one set of helical segments and a plurality of circumferential elements that are joined together by the helical segments to form a stent body. The stent may also have end zones. The combined coverage by these helical windings provides for a uniform stent-to-vessel ratio and hoop strength without detrimentally compromising the expandability of the entire structure.

Also European patent application EP 1.025.812 (Duerig et al.) discloses a stent structure, preferably a self-expanding Nitinol stent, for insertion into a vessel of a patient. The stent is made from a tubular member having a thickness, front and back open ends, and a longitudinal axis extending there between. The member has a first smaller diameter for insertion into a vessel, and a second larger diameter for deployment into a vessel. The tubular member has a plurality of adjacent hoops extending between its front and back ends. The hoops are formed of a plurality of longitudinal struts, each having opposing ends and a centre there between. The ends of the struts are shaped to form a plurality of loops which connect adjacent struts at the ends of the struts. The member further includes a plurality of bridges connecting adjacent hoops to one another. Each of the struts has a width which is greater at its ends than at its centre. Preferably, the width continuously tapers from a greater width at the ends to a smaller width at the centres. This known stent structure, however, does not provide a uniform stent-to-vessel ratio as well as other desired properties.

Another known stent structure is known from International patent application WO 99/44535 (Tseng et al.). This known intra-luminal stent is made of a zigzag or sinusoidal member defining a successive series of struts, connected by apex sections and formed into a series of axially displaced hoop members wherein at least one of the hoop members has at least one strut connected to a strut of an adjacent hoop. The connected struts may be connected by spot welding, continuous welding, or suturing, for example, or by a bridging member connected to each strut, and may be spaced along the length of the stent in a pattern to form a connective spine. The number of zigs of the zigzag member in each hoop member may be varied, as can the zig length. A plurality of connective spines may also be included. This known structure, however, does not comprise helical segments having distinctive quantities of linear segments or struts.

### SUMMARY OF THE INVENTION

The present invention is directed to balloon expandable stents that have relatively uniform stent-to-vessel ratios when expanded and other desirable. A stent of the type as described in the opening paragraph is, in accordance with the present invention, characterized in that the first and second circumferential segments alternate with one another in each cylindrical element; in that the first circumferential segments of adjacent cylindrical elements are offset from each other to form a first expandable helical segment including a plurality of the first circumferential segments alternating with a plurality of the connecting elements; and in that the second circumferential segments of adjacent cylindrical elements are offset from each other to form a second expandable helical segment including a plurality of second circumferential segments alternating with a plurality of the connecting elements, and in that said first helical segment and said second helical segment cross each other in shared connecting elements. The stents of the present invention comprise a generally cylindrically shaped main body having a plurality of expandable helical segments. The main body is comprised of a plurality of cylindrical main body elements that are joined together by the helical segments. The cylindrical elements have cylindrical axes that are collinear with the cylindrical axis of the main body. The cylindrical elements are formed from a plurality of circumferential elements that are joined together by the expandable helical segments. In some embodiments, the stent may comprise endzones that straddle the main body.

In one embodiment of the present invention, the stent may comprise a first non-helical endzone and a second non-helical endzone that straddle the main body. The main body is generally cylindrically shaped and has a cylindrical axis. A plurality of adjacent main body cylindrical elements are connected together to form the main body of the stent. Each main body cylindrical element may be comprised of a plurality of expandable first and second circumferential elements. In some embodiments, the second circumferential elements have a circumferential dimension less than the circumferential dimension of the first circumferential elements. In yet other embodiments, the first and second circumferential elements have the same circumferential dimensions and are substantially identical except that, with respect to the cylindrical axis of the stent, they are oriented differently. Each second circumferential segment in each main body cylindrical element is connected to two first circumferential segments. In addition, each second circumferential segment in each main body cylindrical element is connected to a second circumferential segment in an adjoining main body cylindrical element thereby forming a plurality of helixes in the main body of the stent.

In one embodiment, the main body may be comprised of a plurality of first helical segments each having a substantially identical first pitch and a plurality of second helical segments, each having a substantially identical second pitch. The first and second pitches are generally different. In at least one embodiment, the second pitch is twice that of the first, and at least one first helical segment crosses one of the second helical segments.

The stents of the present invention may be manufactured from a tubular member by removing material from the tube to form a first endzone region, a second endzone region, and a middle region. By removing material from the middle region a plurality of parallel helical segments will remain and a plurality of circumferential segments will remain connecting the helical segments. Alternatively, the stent may be formed from a tube by removing material such that at least two sets of helical segments remain with each set having a different pitch.

### BRIEF DESCRIPTION OF THE DRAWINGS

- Figure 1: is a three dimensional view of one embodiment of a stent according to the present invention in its unexpanded state.
- Figure 2: is planar view of a flattened portion of the circumference of the stent in Figure 1.
- Figure 3: is an enlarged portion of Figure 2.
- Figure 4: is another planar view of a flattened portion of the circumference of a stent according to the present invention in its unexpanded state.
- Figure 5: is an enlarged view of a portion of Figure 4 showing a first circumferential element of the stent.
- Figure 6: is an enlarged view of a portion of Figure 4 showing a second circumferential element of the stent.
- Figure 7: is a planar view of a flattened portion of the stent in Figure 1 showing a plurality of sets of helical segments propagating through the stent's body.
- Figure 8: is a planar view of a flattened endzone that may be employed in a stent of the present invention.
- Figure 9: is a planar view of a flattened portion of part of the endzone shown in Figure 8.

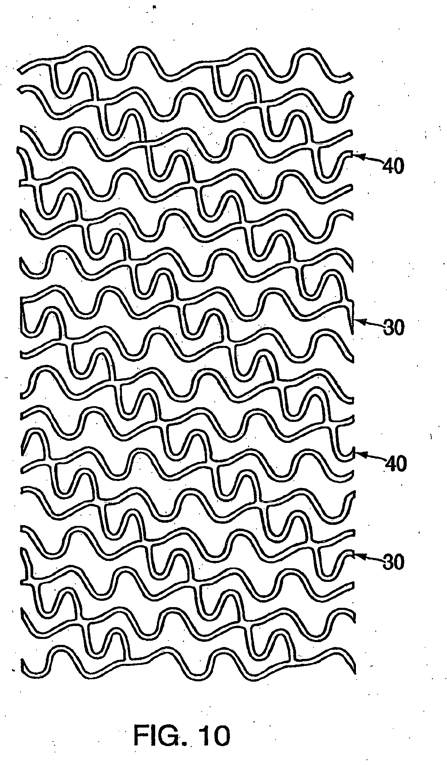

- Figure 10: is a planar view of a flattened portion of an expandable stent according to the prior art, after the stent has been deployed in a lumen.
- Figure 11: is three dimensional view of an alternative embodiment of the present invention.
- Figure 12: is a three dimensional view of a stent.
- Figure 13: is a planar view of the stent shown in 12.
- Figure 14: is a detailed view of a portion of Figure 13.
- Figure 15: is a detailed view of another portion of Figure 13.

### DETAILED DESCRIPTION OF THE INVENTION

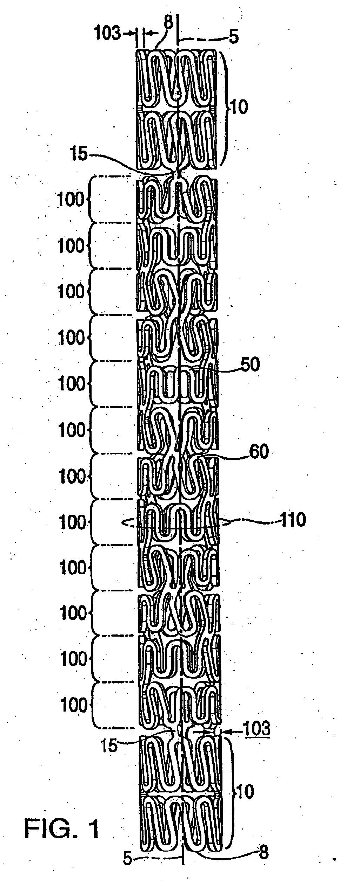

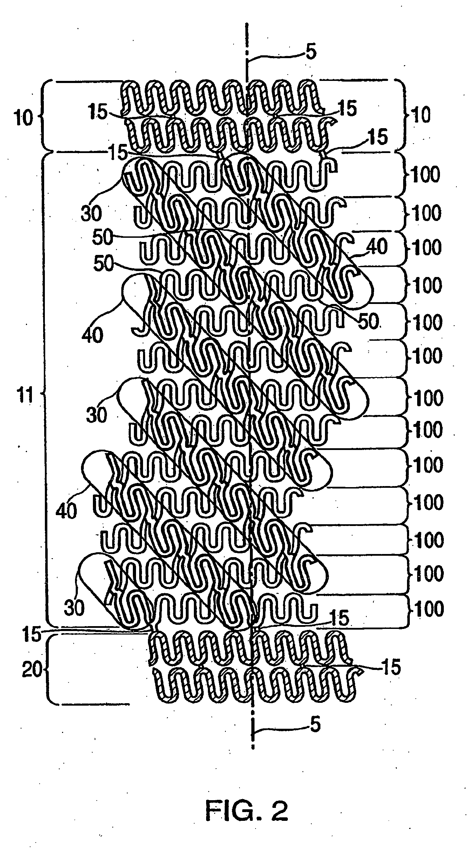

The present invention is directed to an expandable stent, as well as a method of manufacturing the stent. In one embodiment, as is shown in Figures 1 and 2, the stent comprises a generally cylindrical shaped main body section 11 having a cylindrical axis 5 and a wall thickness 103. The wall thickness 103 may optionally be uniform throughout the stent. The main body section 11 is comprised of a plurality of helical segments 30 and 40 and a plurality of main body cylindrical elements 100, each having cylindrical axes (not shown) that are collinear with the main body cylindrical axis 5. The main body cylindrical elements 100 are each comprised of circumferential elements 50 that are joined together by the helical segments 30 and 40 to form individual cylinders 100.

The stent may also have a first endzone 10 and a second endzone 20 that straddle the body section 11. In some embodiments, such as the one shown in Figure 1, the endzones 10 and may advantageously provide the stent with square outer edges 8. The stent may be manufactured from stainless steel, or other suitable materials. In most embodiments, it is desirable that the material, or a portion of the material, be radiopaque and that the various segments that form the stent be contiguous. Although, in some embodiments, the various segments that make up the stent can be distinct elements that are joined together. The main body 11, shown in Figures 1 and 2, may be formed in numerous ways. For example, the body 11 may contain two or more first helical segments 30 and 40 that are generally parallel to each other. In some embodiments they may be opposite each other by 180.° In general, the first helical segments 30 and 40 will be spaced equidistant along the circumference 110 of the main body 11. The first helical segments 30 and 40 are joined by a plurality of circumferential segments 50 to form a plurality of main body cylindrical elements 100, which may be only generally cylindrically shaped. In one embodiment, the circumferential segments 50 make up a majority of the circumference 110 of each cylindrical element 100. In addition to joining the circumferential elements 50 to form cylindrical elements 100, the helical segments 30 and 40 connect each cylindrical element 100 to an adjacent cylindrical element 100 to form the main body 11.

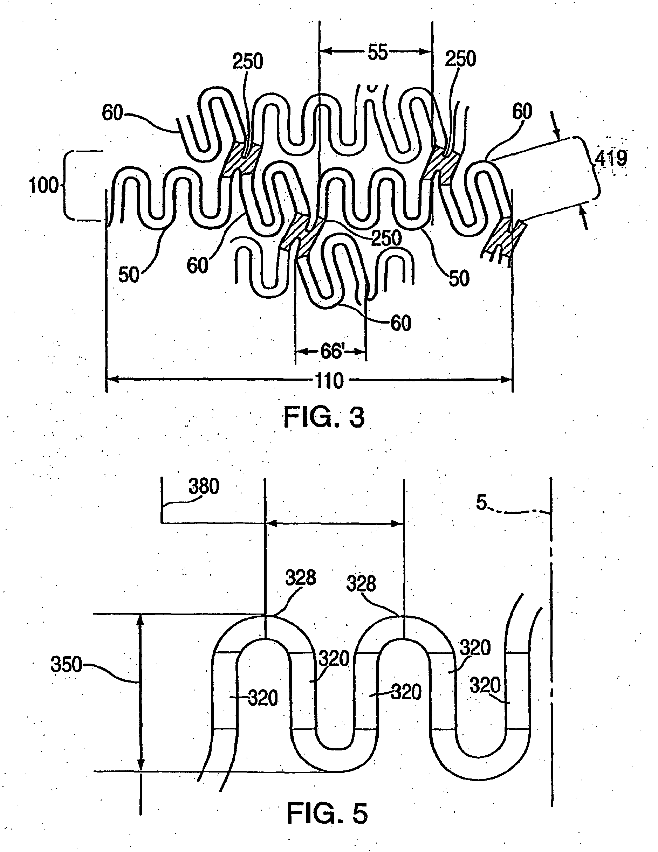

As is shown in Figure 3, the body of the stent 11 comprises a plurality of main body cylindrical elements 100 formed from first circumferential segments 50 that are joined with second circumferential segments 60. The second circumferential segments 60 of each cylindrical element 100 are joined with second circumferential segments 60 of adjacent cylindrical elements 100 to form a plurality of first helical segments 30 and 40 in the main body 11. (See Fig. 2). Each first circumferential segment 50 may have a circumferential dimension 55 and each second circumferential segments 60 may have a circumferential dimension 66. (See Fig.3) In some embodiments, it may be desirable for the circumferential dimension 55 of the first expandable element 50 to be larger than the circumferential dimension 66 of the second expandable element 60.

The first circumferential segment 50 may be an expandable segment formed from plurality of segments joined together to form a pattern. The pattern, such as the one shown in the Figures 1-3, may be a repeating pattern that resembles a square wave form having curved peaks and valleys. Other patterns, both repeating and non-repeating, may be used. For example, and -without limitation, the first circumferential segments 50 may resemble a triangle wave form, a sinusoidal wave form, other repetitious patterns, or any pattern that enables the segment to expand when a radial force is exerted on the stent from the inside or collapse radially when an external crimping force is applied.

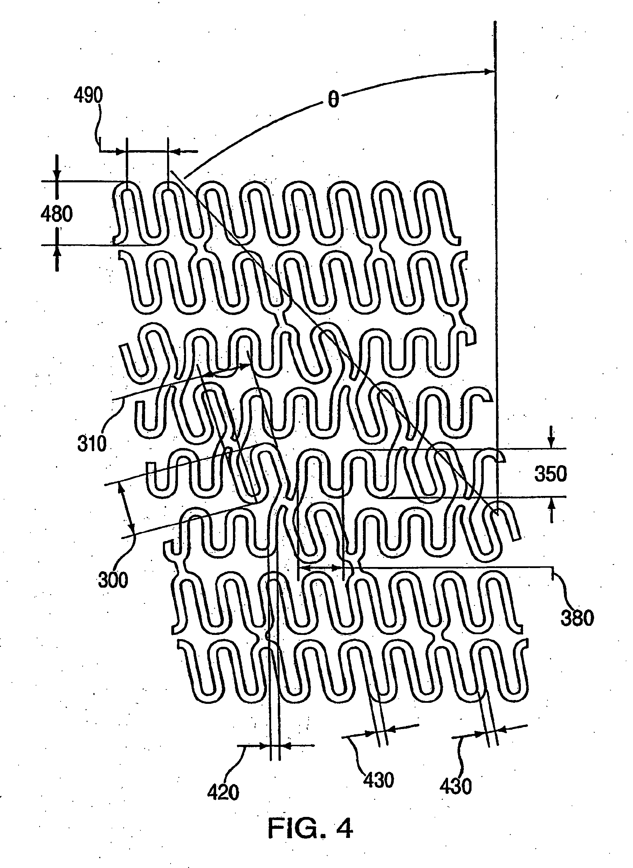

The first circumferential elements 50 may have a filament width 420 (see Fig. 4). In one embodiment, the filament width may vary between 0.051 mm and 0.178 mm, but is preferably about 0.127 mm. Other filament widths may be used depending on the parameters of the stent.

In the embodiment shown in Figs. 1-5, the first circumferential elements 50 comprise linear portions 320 and curved portions 328 that join the linear portions 320 together to form a repeating pattern. In some, but not all, embodiments, the linear portion 320 may be parallel to the cylindrical axis of the stent. The first circumferential segment 50 has an amplitude 350 and a period 380. In one embodiment the amplitude may range from 0.5 mm to 2.0 mm and the period may range from 0.5 mm to 2.0 mm. In some embodiments, the amplitude is less than the period. Other amplitudes and periods may be used depending on the overall stent design and performance constraints.

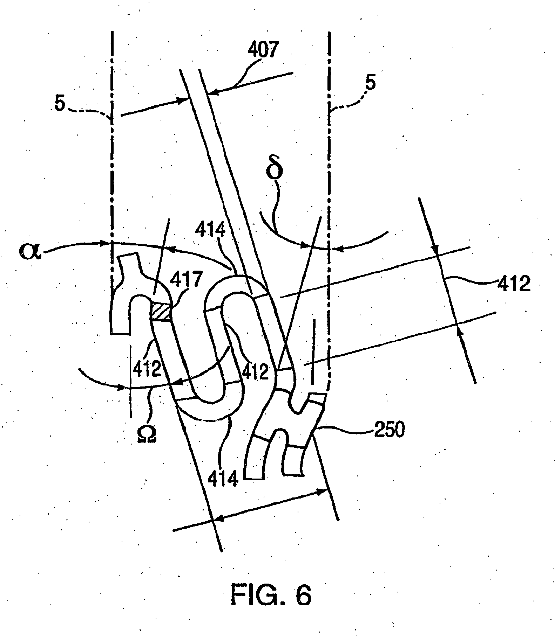

The second circumferential elements 60, which are joined together in a helical pattern to form one or more helical segments 30 or 40, may also take numerous forms, in addition to the form shown in Figure 6. In the embodiment shown in Fig 6, the second circumferential element 60 comprises linear portions 412 and curved portions 414 having a filament width 407, and) resembles generally an S-shaped structure. In addition, the second element circumferential segment 60 may have an angled portion 417 attached to the linear portion 412 at an end opposite that of the curved portion 414. The angled portion may be oriented to form an angle α relative to the cylindrical axis of the stent 5 in the range of 0-45 degrees. In at least one embodiment, the preferable angle α is about 10 degrees. In some embodiments, the linear portions 412 of the second circumferential element 60 lies at an angle Ω relative to the cylindrical axis of the stent, wherein Ω preferably ranges from 0 to 45 degrees. When viewed in a planar fashion as in Fig. 2, the linear portions 412 may, in some embodiments, form an angle Ω, relative to the cylindrical axis of the stent. In some embodiments, Ω may be approximately equal to the helical angle of the first helical segments 30 and 40. In one embodiment, the second circumferential elements 60 may have an amplitude 300 (see Figs. 3, 4, and 6) ranging from 0.5 mm to 2.0 mm and a period ranging from 0.5 mm to 2.0 mm. Other ranges may be used depending on the particular stent size and design being employed. In one embodiment, the preferred period is about 0.82 mm and the preferred length of the linear portion 412 is about 0.5 mm and the amplitude 300 is about 0.38 mm. The amplitude of the second circumferential element 60 may be greater than, equal to, or less than the amplitude of the first circumferential element 50. In one embodiment, the circumferential contributions of the first circumferential elements 50 to the overall circumference of the main body 11 is greater than the circumferential contribution of the second circumferential element 60, in terms of either circumferential length or circumferential cylindrical surface area. In one embodiment, the stent may have an overall outer surface area of about 18.71 square mm.

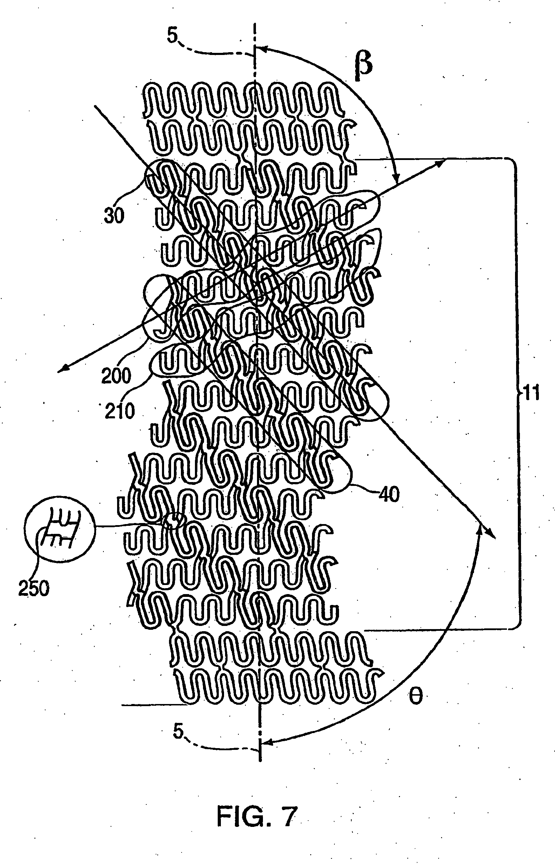

As is shown in Figure 7, the stent has a main body 11 comprising two or more first helical segments 30 and 40, as well as two or more second helical segments 200 and 210. The first and second helical segments 30, 40 and 200, 210, respectively, are joined together to form a generally cylindrically shaped body 11. The first and second helical segments share a common connecting element 250. In some embodiments, the common connecting element 250 may be H-shaped and the two generally parallel linear portions of the H-shaped connecting segment 250 may form an angle δ relative to the axis 5. (See Fig. 6). δ may, in one embodiment, be about 14 degrees. As is shown in Figure 7, the first helical segments 30 and 40 and second helical segments 200 and 210 may have different pitches, i.e. number of spirals per unit length, which results in the first and second helical segments as having different helical angles (θ and β, respectively) i.e. the angle of the helical segment relative to the cylindrical axis 5 of the stent. In one embodiment, the second helical segments 200 and 210 have a pitch approximately twice that of the first helical segments. In one embodiment 8 may vary from 0 to 45 degrees and is preferably about 40 degrees and β is preferably about twice θ. In other embodiments the angle θ may range from 0 to 90 degrees to the circumference 110 of each cylindrical element 100.

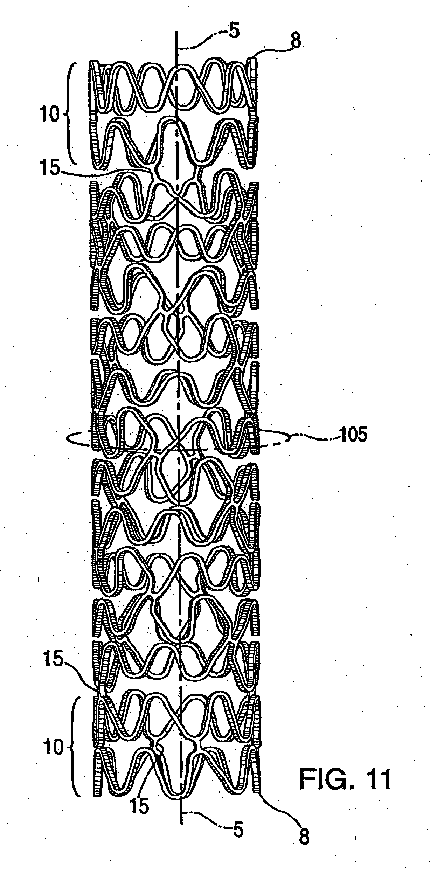

As is shown in Figures 2,3,4, and 6, the helical segments 30,40 are circumferentially expandable (i.e. they expand along the circumference of the stent) and are formed from a plurality of circumferential elements 60 that in turn may be made up of linear 412 and curved 414 segments (see Fig. 6) that each have a filament width 407 (see Fig. 6) that is less than the circumferential dimension 66 of the circumferential element 60 (see Fig. 3). In some embodiments, each helical segment 30 or 40 will make a total contribution to the circumference of each cylindrical element 100 that is greater than the filament width 407. The circumferential contribution of each helical segment 30 or 40 to the overall circumference of the stent (110 in Figure 1 or 105 in Figure 11) may be greater than the circumferential contribution of the filament widths 407 of the segments (e.g. 412 and 414) making up the circumferential elements 60 that in turn make up the helical segments. (I.e., In some embodiments the circumferential contribution of the helical segments 30 and 40 to the circumference 110 of each cylindrical element 100 is more than just a function of the filament width 407, e.g., it may be a function of the geometry of the element 60.) For the embodiment shown in Figures 1 and 11, this is the case when the stent is in both the unexpanded and expanded state. The geometry of the helical segments 30 and 40 are a factor in determining their expandability.

Likewise, the helical segments 200, 210 are circumferentially expandable and are comprised of other circumferential elements 50 that may in turn be comprised of linear 320 and curved segments 328 (see Figs. 3 and 5) that have a filament width 420 (see Fig. 4). The contribution of the helical segments 200,210 to the overall circumferential dimension 110 of each cylindrical element 100 is greater than just the contribution of the filament widths 420 of the individual segments 320 and 328 that make up the elements 50 that in turn make up the helical segments 200, 210. The geometry of the elements 50 making up the helical segments 200, 210 may be a more important factor in determining the circumferential contribution of the helical segments 200 and 210 to the overall stent circumference than the filament width 420. Thus, in one embodiment of the present invention, the circumference of the stent 110 in its unexpanded state and the circumference 105 when the stent is expanded are primarily functions of the geometry of the elements 50 and 60, that make up the helical segments 30, 40 and 200, 210, respectively.

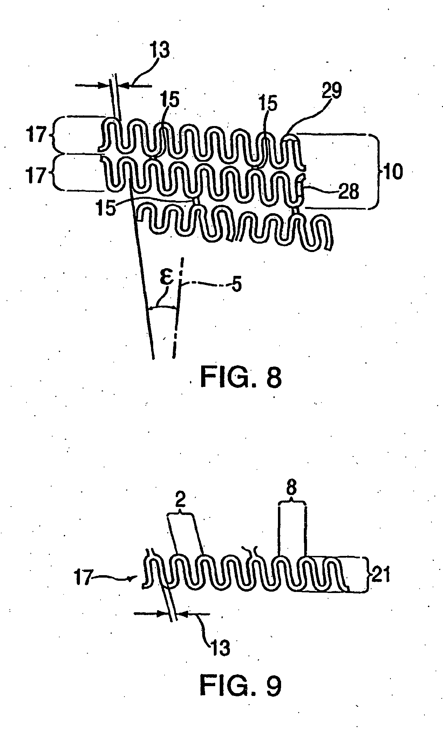

Some, but not all embodiments, of the present invention may employ endzones 10 and 20. (See Figs. 1,2, and 11). Stents that employ endzones will generally have two endzone regions straddling a central zone in the middle of the stent The stents may also have a transition region between the endzone and the central zone. The transition region serves to help smoothly transition between the expanded middle region and an portions of the end of the stent that remain unexpanded after the stent is implanted. The size and characteristics of the transition region are a function of the material and geometry of the stent. For example, the transition range properties vary as a function of, among other things, the helical angle of the first helical segments, the number of curved segments located in the endzones, and the angle e of the linear portions of the segments forming the endzones. (See e.g. Fig. 8).

The endzones 10 and 20 may take numerous forms. In some embodiments, the endzones may be comprised of one or more rings 17. (See Fig. 8). The rings 17 may be generally cylindrically shaped, and in some embodiments, right cylindrically shaped. In one embodiment, the rings are formed from linear segments 28 joined together by curved segments 29 to form a pattern. The pattern, which is preferably- but not necessarily - a repeating pattern may take numerous forms, including the one shown. The endzones 10 and 20 may be comprised of a plurality of rings 17 attached together. Struts 15 may be used to attach the rings together to form the endzone and to attach the endzone to the main body 11. The struts, in some embodiments, act as cantilever springs and there stiffness, which is a function of their width and thickness, may define bending properties of the stent along its cylindrical axis 5.

In the embodiment shown in Figs. 1, 7, 8, and 9, which is exemplary only, the linear segments 28 in the endzone 10, are oriented at an angle ε relative to the cylindrical axis of the stent. The angle ε may range from 0 to 45 degrees and in one embodiment is preferably about 10 degrees. The segments of the endzone may have a filament width 13 of between 0.051 and 0.178 mm. In one embodiment, the repeating pattern of the endzone has a period 2 of about 0.686 mm and an amplitude 21 of about 1.092 mm. Other values may be used. As is shown in Figure 1, the struts 15, which are but one way to attach the endzones 10 and 20 to the main body 11, may, in one embodiment have a width of between 0.051 mm and 2.032 mm and preferably the width does not exceed the wall thickness, which typically but not necessarily - ranges from about 0.051 to 0.203 mm.

The stent of the present invention may, after insertion into a vessel, be expanded such that it plastically deforms from the unexpanded state to an expanded state having a diameter increase of about 400 to 500%, which results in a large circumference 105. (See Figure 11). Figure 11 depicts the stent shown in Figure 1 in an expanded state. Upon expansion the stent's outer diameter in one particular embodiment increases from 1.0 mm to 3.00 mm and maintains a stent-to-vessel ratio in the expanded state that is greater than on average 16%.

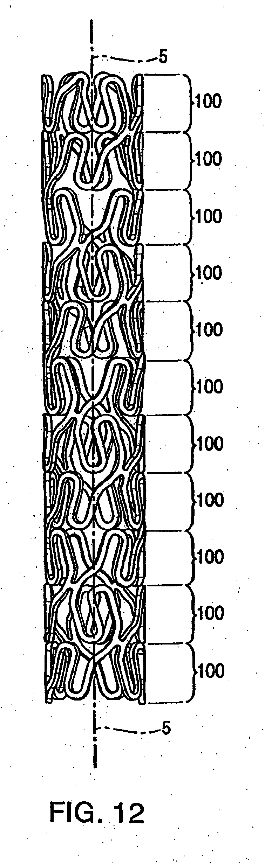

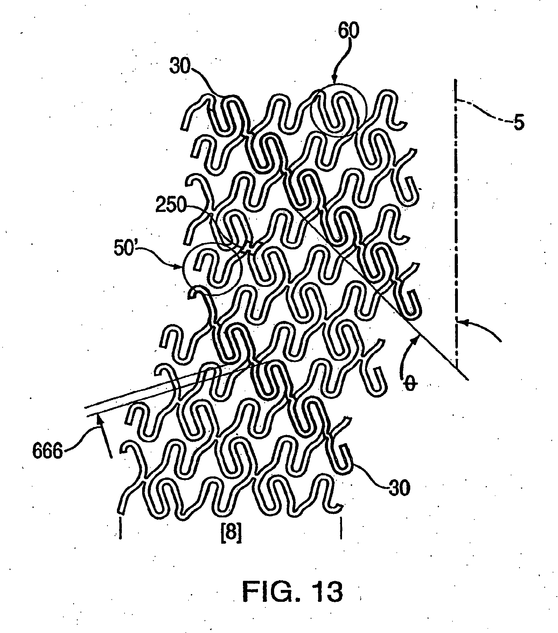

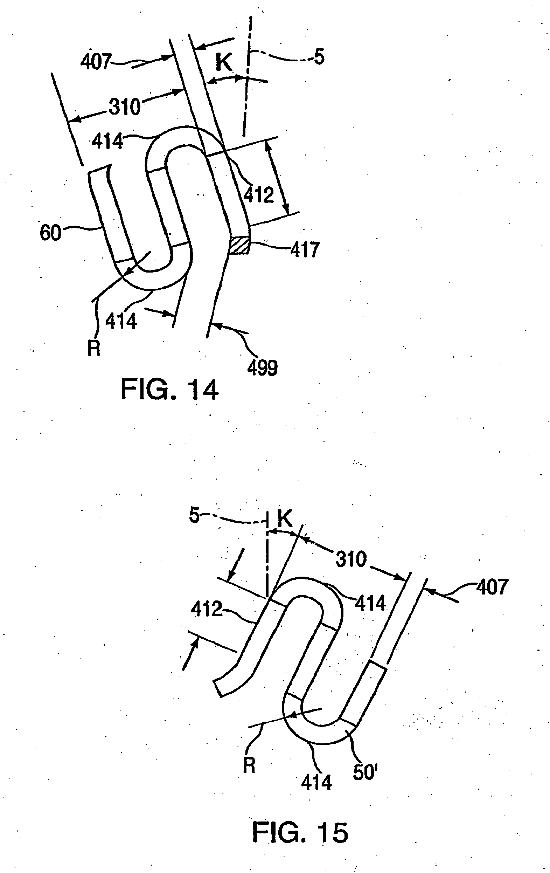

While endzones 10 and 20 may be used to provide square edge, not all stents according to the present invention require endzones. Figures 12-15 depict an endzoneless stent. Like the stent shown in Figures 1-9, the stent of Figures 12-15, comprises a plurality of adjacent cylindrical elements 100. The cylindrical elements 100 are formed from a plurality of first circumferential elements 50' and second circumferential elements 60. The first circumferential elements 50' of the stent in Figures 12-15 are substantially identical to the second circumferential element 60 except that they are rotated to have a different orientation. The circumferential elements may be generally S-shaped having a linear portion 412, a curved portion 414 having a radius R, and an angled portion 417. R may vary widely depending on overall stent characteristics and in one embodiment varies between 0.025 and 0.051 mm and is preferably about 0.211 mm. The angled portion 417 is spaced a distance 499 from the linear portion. In one particular embodiment, the distance 499 may vary from 0.051 to 0.508 mm and is preferably about 0.178 mm. The filament width 407 of the elements may, in one embodiment, be about 0.13 mm.

Adjacent cylindrical elements 100 are joined together by connecting first circumferential elements 50' in each cylindrical element 100 with first circumferential elements 50' in an adjacent cylindrical element 100, such that the first circumferential elements 50' in adjacent cylindrical elements 100 form helixes through the stent and such that second circumferential elements form helixes through the stent having an angle 9 relative to the axis 5. In some embodiments, a connecting segment 250 (see Fig. 7) is used to connect first circumferential elements in adjacent cylindrical elements 100 and to connect second circumferential elements 60 in adjacent cylindrical elements 100. In addition, the connecting segment, connects first circumferential elements 50' in each cylindrical element 100 with two second circumferential elements 60 in each cylindrical element 100. In one embodiment, the individual cylindrical elements 100 are adjacent to each other and are located a distance 666 apart. In one embodiment, the preferred may range between 0.051 and 0.508 mm, and is preferably about 0.229 mm.

The above description of the stent of the present invention is illustrative and not exhaustive. Various modifications may be made to the stent to change its overall characteristics without deviating from the scope of the invention as defined by the claims. For example and without limitation, the increasing the length of the linear segments and or increasing the arc of the second circumferential elements 60 will decrease the amount of radial force required to expand each circular section and will increase flexibility. Increasing the angle Ω of the second circumferential element 60 will: (I) increase the amount of radial force required for expansion, (ii) increase surface area, and (iii) decrease flexibility. Likewise, various modifications may be made to the struts 15. (See Fig.2). Increasing strut width and wall thickness will: (I) increase surface area, (ii) increase radial strength, (iii) increase pressure required to expand the stent radially, (iv) decrease flexibility, and, in the case of increased wall thickness, (v) increase radiopacity.

The stent of the present invention may be manufactured in numerous ways. The stent may be formed from a metallic tube by removing various portions of the tube's wall to form the patterns described herein. The resulting stent will thus be formed from a single contiguous piece of material, eliminating the need for connecting various segments together. Material from the tube wall may be removed using various techniques including laser (YAG laser for example), electrical discharge, chemical etching, metal cutting, a combination of these techniques, or other well known techniques. See e.g. U.S Patent Nos. 5,879,381 to Moriuchi et al. and 6,117,165 to Beaker. Forming stents in this manner allows for creation of a substantially stress-free structure where the helical segments are integral with the circumferential elements. In one embodiment, the tube from which the stent is formed may have an internal diameter of about 3.0 mm, a wall thickness of about 1.0 mm and a length of about 30 mm. Tubes having other dimensions may be used. In particular, the length may be adapted to that of the diseased part of the lumen in which the stent is to be placed. This may avoid using separate stents to cover the total diseased area.

Those skilled in the art will recognize that the stent described above is illustrative and not exhaustive of the present invention and that modifications and variations maybe made without deviating from the scope of the invention as defined by the following claims.

## Claims

1. Balloon expandable stent comprising at least one helical segment (30, 40, 200, 210), and comprising a plurality of adjacent cylindrical elements (100) connected to one another by a plurality of connecting elements (250), in which the cylindrical elements (100) have cylindrical axes that are collinear with the stent's cylindrical axis and each cylindrical element (100) includes a plurality of first circumferential segments (50), each first circumferential segment (50) extending between two connecting elements (250) and having a number of linear portions (320), wherein said number is exactly five, and a plurality of second circumferential segments (60), each second circumferential segment (60) extending between two connecting elements (250) and resembling generally an S-shaped structure having a number of linear portions (412), wherein said number is exactly three, **characterized in that** the first and second circumferential segments (50, 60) alternate with one another in each cylindrical element (100); **in that** the first circumferential segments (50) of adjacent cylindrical elements (100) are offset from each other to form a first expandable helical segment (200, 210) including a plurality of the first circumferential segments (50) alternating with a plurality of the connecting elements (250); and **in that** the second circumferential segments (60) of adjacent cylindrical elements (100) are offset from each other to form a second expandable helical segment (30, 40) including a plurality of second circumferential segments (60) alternating with a plurality of the connecting elements (250), and **in that** said first helical segment and said second helical segment cross each other in shared connecting elements.

2. Stent according to claim 1, wherein the cylindrical elements (100) are cylindrical rings; each cylindrical ring is defined by a plurality of first circumferential segments (50) alternating with a plurality of second circumferential segments (60), each first circumferential segment (50) including five linear struts (15) positioned between two connecting elements (250), and each second circumferential segment (60) including three linear struts (15) positioned between two connecting elements (250); the first helical segment (30, 200) is defined by a plurality of the first circumferential segments (50) alternating with a plurality of the connecting elements (250); and the second helical segment (40, 210) is defined by a plurality of the second circumferential segments (60) alternating with a plurality of the connecting elements (250).

3. Stent according to claim 1 or 2, wherein the connecting elements (250) are spaced apart by a plurality of first circumferential segments (50) including five linear struts (15) and a plurality of second circumferential segments (60) including three linear struts (15) in an alternating pattern to define the first helical segment (30, 200) having a plurality of the first circumferential segments (50) alternating with a plurality of the connecting elements (250) and the second helical segment (40, 210) having a plurality of the second circumferential segments (60) alternating with a plurality of the connecting elements (250).

4. Stent according to any of the preceding claims, wherein the stent is in an unexpanded state.

5. Stent according to any of the preceding claims, wherein the stent is in an expanded state.

6. Stent according to any of the preceding claims, wherein the linear portions (320) of the first circumferential segment (50) are connected to each other by curved portions (328), and the linear portions (412) of the second circumferential segment (60) are connected to each other by curved portions (414).

7. Stent according to claim 6, wherein the curved portions (328, 414) of at least two adjacent cylindrical elements are circumferentially offset from one another.

8. Stent according to claim 6 or 7, wherein the curved portions of at least two adjacent cylindrical rings are circumferentially staggered from each another.

9. Stent according to any of the preceding claims, comprising at least one endzone connected to one of the cylindrical rings.

10. Stent according to any of the preceding claims, comprising first and second endzones straddling a main body of the stent.

11. Stent according to claim 9 or 10, wherein the endzone comprises a plurality of rings (17) joined together by a plurality of struts (15).

12. Stent according to any of the preceding claims, wherein the stent defines a cylindrical axis (5) and at least one of the connecting segments (250) is inclined with respect to the cylindrical axis (5).

## Patentansprüche

1. Ballon expandierbarer Stent mit zumindest einem helikalen Segment (30,40,200,210) sowie mit einer Vielzahl an aufeinanderfolgenden zylindrischen Elementen (100), die miteinander durch eine Vielzahl an Verbindungselementen (250) verbunden sind, in welchem die zylindrischen Elemente (100) zylindrische Achsen aufweisen, die mit der zylindrischen Achse des Stents kollinear sind, und jedes zylindrische Element (100) eine Vielzahl an ersten umlaufenden Segmenten (50) mit einschließt, wobei sich jedes erste umlaufende Segment (50) zwischen zwei Verbindungselementen (250) erstreckt und eine Anzahl von lineare Segmente (320) hat, wobei die genannte Anzahl genau fünf ist, sowie eine Vielzahl an zweiten umlaufenden Segmenten (60), wobei jedes zweite umlaufende Segment (60) sich zwischen zwei Verbindungselementen (250) erstreckt und im allgemeinen eine S-förmige Struktur mit einer Anzahl von lineare Segmente (412) aufweist, wobei die genannte Anzahl genau drei ist, **dadurch gekennzeichnet, dass** sich die ersten und zweiten umlaufenden Segmente (50, 60) in jedem zylindrischen Element (100) miteinander abwechseln; dass die ersten umlaufenden Segmente (50) der aufeinanderfolgenden zylindrischen Elemente (100) gegeneinander versetzt sind, um ein erstes helikales Segment (200, 210) mit einer Vielzahl der ersten umlaufenden Segmenten (50) zu bilden, die sich mit einer Vielzahl der Verbindungselemente (250) abwechseln; und dass die zweiten umlaufenden Segmente (60) der benachbarten zylindrischen Elemente (100) gegeneinander versetzt sind, um ein zweites helikales Segment (30, 40) mit einer Vielzahl an zweiten umlaufenden Segmenten (60) zu bilden, die sich mit einer Vielzahl der Verbindungselemente (250) abwechseln, und dass das erste helikales Segment und das zweite helikales Segment einander in gemeinsamen Verbindungselementen kreuzen.

2. Stent nach Anspruch 1, in welchem die zylindrischen Elemente (100) zylindrische Ringe sind; jeder zylindrische Ring durch eine Vielzahl an ersten umlaufenden Segmenten (50) definiert ist, welche sich mit einer Vielzahl an zweiten umlaufenden Segmenten (60) abwechseln, wobei jedes erste umlaufende Segment (50) fünf lineare Streben (15) einschließt, die zwischen zwei Verbindungselementen (250) liegen, und jedes zweite umlaufende Segment (60) drei lineare Streben (15) mit einschließt, die zwischen zwei Verbindungselementen (250) liegen; das erste helikale Segment (30, 200) durch eine Vielzahl der ersten umlaufenden Segmente (50) definiert ist, die sich mit einer Vielzahl der Verbindungselemente (250) abwechseln; und das zweite helikale Segment (40, 210) durch eine Vielzahl der zweiten umlaufenden Segmente (60) definiert ist, welche sich mit einer Vielzahl der Verbindungselemente (250) abwechseln.

3. Stent nach Anspruch 1 oder 2, in welchem die Verbindungselemente (250) durch eine Vielzahl an ersten umlaufenden Segmenten (50) mit fünf linearen Streben (15) sowie eine Vielzahl an zweiten umlaufenden Segmenten (60) mit drei linearen Streben (15) in einem abwechselnden Muster räumlich voneinander getrennt sind, um das erste helikale Segment (30, 200) zu definieren, das eine Vielzahl der ersten umlaufenden Segmente (50) besitzt, die sich mit einer Vielzahl der Verbindungselemente (250) abwechseln, und um das zweite helikale Segment (40, 210) zu definieren, das eine Vielzahl der zweiten umlaufenden Segmente (60) besitzt, die sich mit einer Vielzahl der Verbindungselemente (250) abwechseln.

4. Stent nach einem der vorstehenden Ansprüche, in welchem der Stent in einem unexpandierten Zustand ist.

5. Stent nach einem der vorstehenden Ansprüche, in welchem der Stent in einem expandierten Zustand ist.

6. Stent nach einem der vorstehenden Ansprüche, in welchem die linearen Abschnitte (320) des ersten umlaufenden Segments (50) durch gekrümmte Abschnitte (328) miteinander verbunden sind, und dass die linearen Abschnitte (412) des zweiten um-laufenden Segments (60) durch gekrümmte Abschnitte (414) miteinander verbunden sind.

7. Stent nach Anspruch 6, in welchem die gekrümmten Abschnitte (328, 414) mindestens zweier benachbarter zylindrischer Elemente umlaufend versetzt gegeneinander sind.

8. Stent nach Anspruch 6 oder 7, in welchem die gekrümmten Abschnitte mindestens zweier benachbarter zylindrischer Ringe umlaufend gestaffelt gegeneinander sind.

9. Stent nach einem der vorstehenden Ansprüche mit zumindest einem Endbereich, der mit einem der zylindrischen Ringe verbunden ist.

10. Stent nach einem der vorstehenden Ansprüche mit ersten und zweiten Endbereich, die den Hauptkörper des Stents überspannen.

11. Stent nach Anspruch 9 oder 10, in welchem der Endbereich eine Vielzahl an Ringen (17) aufweist, die miteinander durch eine Vielzahl an Streben (15) miteinander verbunden sind.

12. Stent nach einem der vorstehenden Ansprüche, in welchem der Stent eine zylindrische Achse (5) definiert, und wobei zumindest eines der Verbindungselemente (250) bezüglich der zylindrischen Achse (5) schräg ist.

## Revendications

1. Stent ballon expansible comprenant au moins un segment hélicoïdal (30, 40, 200, 210), et comprenant une pluralité d'éléments cylindriques (100) adjacents raccordés entre eux par une pluralité d'éléments de raccordement (250), dans lequel les éléments cylindriques ont des axes cylindriques colinéaires avec l'axe cylindrique du stent, et chaque élément cylindrique (100) comprend une pluralité de premiers segments circonférentiels (50), chaque premier segment circonférentiel (50) s'étendant entre deux éléments de raccordement (250) et ayant une nombre de segments linéaires (320), dans lequel ledit nombre est exactement cinq, et une pluralité de seconds segments circonférentiels (60), chaque second segment circonférentiel (60) s'étendant entre deux éléments de raccordement (250) et ressemblant généralement à une structure en forme de S ayant une nombre de segments linéaires (412), dans lequel ledit nombre est exactement trois, **caractérisé en ce que** les premiers et seconds segments circonférentiels (50, 60) alternent les uns avec les autres dans chaque élément cylindrique (100) ; **en ce que** les premiers segments circonférentiels (50) d'éléments cylindriques (100) adjacents sont décalés les uns par rapport aux autres afin de former un premier segment hélicoïdal (200, 210) comprenant une pluralité de premiers segments circonférentiels (50) alternant avec une pluralité d'éléments de raccordement (250) ; et **en ce que** les seconds segments circonférentiels (60) des éléments cylindriques (100) adjacents sont décalés les uns par rapport aux autres afin de former un second segment hélicoïdal (30, 40) comprenant une pluralité de seconds segments circonférentiels (60) alternant avec une pluralité d'éléments de raccordement (250), et **en ce que** ledit premier segment hélicoïdal et ledit second segment hélicoïdal se croisent dans des éléments de connexion partagés.

2. Stent selon la revendication 1, dans lequel les éléments cylindriques (100) sont des anneaux cylindriques ; chaque anneau cylindrique est défini par une pluralité de premiers segments circonférentiels (50) alternant avec une pluralité de seconds segments circonférentiels (60), chaque premier segment circonférentiel (50) comprenant cinq entretoises linéaires (15) positionnées entre deux éléments de raccordement (250), et chaque second segment circonférentiel (60) comprenant trois entretoises linéaires (15) positionnées entre deux éléments de raccordement (250) ; le premier segment hélicoïdal (30, 200) est défini par une pluralité de premiers segments circonférentiels (50) alternant avec une pluralité d'éléments de raccordement (250) ; et le second segment hélicoïdal (40, 210) est défini par une pluralité de seconds segments circonférentiels (60) alternant avec une pluralité d'éléments de raccordement (250).

3. Stent selon la revendication 1 ou 2, dans lequel les éléments de raccordement (250) sont espacés par une pluralité de premiers segments circonférentiels (50) comprenant cinq entretoises linéaires (15) et une pluralité de seconds segments circonférentiels (60) comprenant trois entretoises linéaires (15) selon un modèle alterné afin de définir le premier segment hélicoïdal (30, 200) ayant une pluralité de premiers segments circonférentiels (50) alternant avec une pluralité d'éléments de raccordement (250) et le second segment hélicoïdal (40, 210) ayant une pluralité de seconds segments circonférentiels (60) alternant avec une pluralité d'éléments de raccordement (250).

4. Stent selon l'une quelconque des revendications précédentes, dans lequel le stent est dans un état non expansé.

5. Stent selon l'une quelconque des revendications précédentes, dans lequel le stent est dans un état expansé.

6. Stent selon l'une quelconque des revendications précédentes, dans lequel les parties linéaires (320) du premier segment circonférentiel (50) sont raccordées entre elles par des parties incurvées (328), et les parties linéaires (412) du second segment circonférentiel (60) sont raccordées entre elles par des parties incurvées (414).

7. Stent selon la revendication 6, dans lequel les parties incurvées (328, 414) d'au moins deux éléments cylindriques adjacents sont circonférentiellement décalées les unes des autres.

8. Stent selon la revendication 6 ou 7, dans lequel les parties incurvées d'au moins deux anneaux cylindriques adjacents sont circonférentiellement échelonné l'une par rapport à l'autre.

9. Stent selon l'une quelconque des revendications précédentes, comprenant au moins une zone d'extrémité raccordée à l'un des anneaux cylindriques.

10. Stent selon l'une quelconque des revendications précédentes, comprenant des première et seconde zones d'extrémité chevauchant un corps principal du stent.

11. Stent selon la revendication 9 ou 10, dans lequel la zone d'extrémité comprend une pluralité d'anneaux (17) assemblés par une pluralité d'entretoises (15).

12. Stent selon l'une quelconque des revendications précédentes, dans lequel le stent définit un axe cylindrique (5) et au moins l'un des segments de raccordement (250) est incliné par rapport à l'axe cylindrique (5).
